# EUROPEAN PATENT SPECIFICATION

(11) **EP 3 143 950 B1**
(45) Date of publication and mention of the grant of the patent: **10.10.2018**
(21) Application number: 15821395.9
(22) Date of filing: 21.01.2015
(51) Int. Cl.: A61B 17/22

(54) **FILM-TYPE URETER STONE BLOCKAGE EXTRACTOR**
FOLIENARTIGER EXTRAKTOR FÜR HARNLEITERSTEINBLOCKADE
APPAREIL D'EXTRACTION DE TYPE MEMBRANE DE CALCULS URÉTÉRAUX D'OBSTRUCTION

(30) Priority: 15.07.2014 CN 201410334431
(43) Date of publication of application: 22.03.2017
(73) Proprietor: Second Military Medical University of the People's Liberation Army, Shanghai 200433 (CN)
(72) Inventor: SUN, Yinghao, Shanghai 200433 (CN)
(74) Representative: Valea AB
(86) International application number: PCT/CN2015/071175
(87) International publication number: WO 2016/008291

(56) References cited:
- WO-A2-2009/086512
- CN-A- 101 554 343
- CN-A- 104 095 665
- CN-U- 203 988 234
- DE-A1- 3 438 131
- DE-A1- 3 607 295
- US-A- 3 472 230
- US-A- 4 406 653
- US-A1- 2002 058 904
- US-A1- 2007 203 475
- US-A1- 2010 137 846

## Description

### TECHNICAL FIELD

The present invention relates to medical devices. More specifically, the present invention relates to a film-type ureter stone blockage extractor.

### BACKGROUND

The incidence rate of urinary stone is up to 5%-10%, which may occur at any part of kidney, bladder, ureter and urethra. Among which, kidney and ureteral stone is the most common. Ureteroscopic lithotripsy for the treatment of urinary stone fragments within the natur3l human body lumen. It is a kind of minimally invasive treatment and can accurately fragment stone, so it is currently one of the main treatment means. However, current ureteroscopic lithotripsy has some deficiencies: 1. stone and stone fragments in the intermediate segment and upper segment of ureter can be easily flushed back to kidney by the recoil force of the injected fluid or the fragmenting tool; 2. The residual stone fragments can't be extracted out efficiently.

An important means for preventing ureteral stone from being flushed back to the kidney is to occlude the upper side of ureteral stone by tools. There are some clinic ureter occluding device which also can be used to extract stone. However, they still have shortcomings.

Basket is commonly used for occluding and extracting stone. The basket will expand as a mesh after moving across to the upper side of the stone, which will prevent the stone fragments from drifting upward and grasp the small stone fragments. But the basket is of large volume and is not convenient to be introduced into ureter. It may push the stone fragments to drift. Meanwhile, the basket need to be repeatedly introduced into the ureter as only a small amount of stone can be extracted at a once, and there is risk for residue stone to dislodge when the water was injected again and again. In addition, sharp stone can be easily pushed out through the holes of basket and scratch the ureter wall during dragging procedure, and may bring complication.

Another occluding method is to use film or balloon to occlude the ureter lumen as much as possible. For example, Chinese Patent CN 200910057068.2, published on Oct 14, 2009, discloses an occluding device for occluding obstacles in body lumen. The occluding device comprises a guide wire, a catheter and an occluding object. One distal end of the guide wire passes through the catheter and the body lumen. The distal end is fixed to the guide wire. Pull the proximal end of the guide wire, the expanded occluding object is axially compressed to an emboliform occluding object. The occluding device is flat film. The occluding device prevents stone from moving upwards by forming emboliform occluding object, and the occluding device can be used for dragging a plurality of stone fragments towards the bladder after the surgery. However, the occluding device can't totally occlude the ureter lumen, especially during the stone dragging process, because the occluding device is prone to deform due to water injection into the working channel of the endoscope. Thus, stone will slip via the space between the occluding device and the ureter wall, or stone and the occluding device may be trapped in the ureter and the ureter mucosa will be injured if there are large quantity of stone. Furthermore, the existing film-like occluding device or balloon often has large volume, so it is not easy to be introduced into ureter like basket, and is more easily be pushed back to kidney.

Documents WO 2009/086512; US 4,406,653 and DE 3438132 disclose balloon catheters for use in endoscopic foreign body extractions.

Document US 2002/0588904 discloses a drug delivery and thrombus removal device comprising: a shaft, having two lumens including a guidewire/drug delivery lumen and a thrombus removal lumen; and a clot collection basket.

As mentioned above, there is an urgent need for a ureter stone blockage extractor which can fully occlude the ureter stone, convenient to extract stone, not easy to deform when crushed stone is being removed, no injury with be made to the ureter wall, can be conveniently introduced into ureter, not easy to cause dislodges of stone. But there is no such kind of devices been reported at present.

### SUMMARY

To overcome deficiencies in the prior art, a film-type ureter stone blockage extractor is provided.

According to one embodiment of the present invention, a film-type ureteral stone blockage, as defined in appended claim 1, is provided with a hollow operating rod that has an open proximal end and a closed distal end, wherein, an occluding film is provided on a distal end segment of the operating rod; during use of the extractor, viewing from one end of the operating rod to the other end thereof, the occluding film is configured as a round occluding object; a distance from a free side of the occluding film to the distal end of the operating rod is less than a distance from a fixed side of the occluding film to the proximal end of the operating rod; a through-hole is also provided in a wall of the operating rod, wherein water stream flowing from a lumen of the operating rod through the through-hole exerts force on an inner wall of the occluding object; and supporters are provided in between the occluding film and the operating rod, the supporters are attached to both the inner wall of the occluding film and the outer wall of the operating rod to support the occluding film.

In one preferred embodiment, the distal end of the operating rod is provided with a fitting groove, the occluding film is provided in the fitting groove, the through-hole is provided on the wall of the fitting groove.

In another preferred embodiment, the occluding film is a single piece of film, the outer surface of the occluding film is curved when it is expanded under normal usage.

The supporters are configured to surround the operating rod and are radially configured with equal space intervals, the through-hole is configured between adjacent supporters.

In another preferred embodiment, comprising a plurality of the occluding films, each of the occluding films is configured to expand as a fan shape with curved surface during use of the extractor, and the occluding films are configured to surround the operating rod in interlocked and overlapped arrangement.

Reinforced film element is provided on the operating rod in between the adjacent occluding films, the reinforced film element surrounds the operating rod, and the fixed side of the occluding film is fixed on the proximal end of the reinforced film element.

Each of two lateral edges of the occluding film and a fixed side of the occluding film is arranged to form an included angle area, the two included angle areas are close to each other, and clad to the wall of the operating rod.

In another preferred embodiment, the occluding film is configured to expand as spiral shape during use of the extractor, and to surround and to be fixed to the wall of the operating rod.

The occluding film surrounding the operating rod by one circle is defined as one occluding film sub unit, all the sub units have the same or different diameters.

The advantages of the present invention:
1. The occluding object of the ureteral stone blockage extractor of the present invention is film shaped occluding object. The occluding film is of small volume, it is easy to be introduced into the ureter, the stone can't be pushed back to the kidney, and is not prone to injure the ureter wall. The occluding film is soft, so the diameter of the occluding film can be designed to be slightly larger than the diameter of the ureter. Thus, the ureter lumen can be fully occluded to prevent stone from slipping and missing, and stone can be extracted by one time. Furthermore, the incidence of concentration can be reduced.
2. A through-hole is provided on the operating rod, the water stream that are infused from the proximal end of the operating rod to the body lumen and then flows through the through-hole exerts force to the inner wall of the occluding object, thus the force exerted to the occluding film that comes from the water stream infused through the working channel of the endoscope is neutralized, which can effectively prevent the occluding film from turning over and prevent the stone from slipping and missing.
3. The distance from a free side of the occluding film to the proximal end of the operating rod is less than the distance from a fixed side of the occluding film to the proximal end of the operating rod, so that the occluding film can form a space which can contain parts of stone, reduce the friction between the stone fragments and the ureter. Meanwhile, it can prevent the occluding film from turning over during surgical procedure.
4. A fitting groove is provided on the operating rod, the occluding film is provided in the fitting groove, so that the extractor of the invention will have comparatively small diameter, is easy to be introduced into the ureter and prevent the stone from being pushed toward the kidney, and eliminate trauma to the inner wall of the ureter.

### BRIEF DESCRIPTION OF THE DRAWINGS

Figure 1 is a structure diagram of one embodiment of single valve film type ureteral stone blockage extractor.
Figure 2 is an enlarged cutaway view of A in Figure 1.
Figure 3 is a structure diagram of one embodiment of single valve film type ureteral stone blockage extractor before the extractor is used.
Figure 4 is a structure diagram of one embodiment of multiple valves film type ureteral stone blockage extractor.
Figure 5 is an enlarged cutaway view of B in Figure 4.
Figure 6 is the front view of a multiple valves film type ureteral stone blockage extractor.
Figure 7 is a structure diagram of one embodiment of spiral film type ureteral stone blockage extractor.
Figure 8 is an enlarged cutaway view of C in Figure 7.

### DETAILED DESCRIPTION

As used in this disclosure, the term "proximal end" means the nearer end to the operator during the surgical procedure, the term "distal end" means the farer end to the operator during the surgical procedure. The term "proximal end segments of the operating rod 11" means the segment that is near the proximal end, and the term "distal end segment of the operating rod 12" means the segment that is near the distal end.

Detailed description according to the figures will be given combined with embodiments.

Labels and components related to the figures are as follows:

| | |
|---|---|
| 1. Operating rod | 11. Proximal end segments of the operating rod |
| 12. Distal end segment of the operating rod | 2. Handle |
| | |
| 3. Guiding tip | 4. Fitting groove |
| 5. Occluding film | 6. Supporter |
| 7. Through-hole | 8. Reinforced film element |
| 51. Fixed side of the occluding film | 52. Free side of the occluding film |
| 53. Lateral side | 54. Included angle area |
| 55. Occluding film sub unit | |

### Embodiment 1 Single valve film type ureteral stone blockage extractor

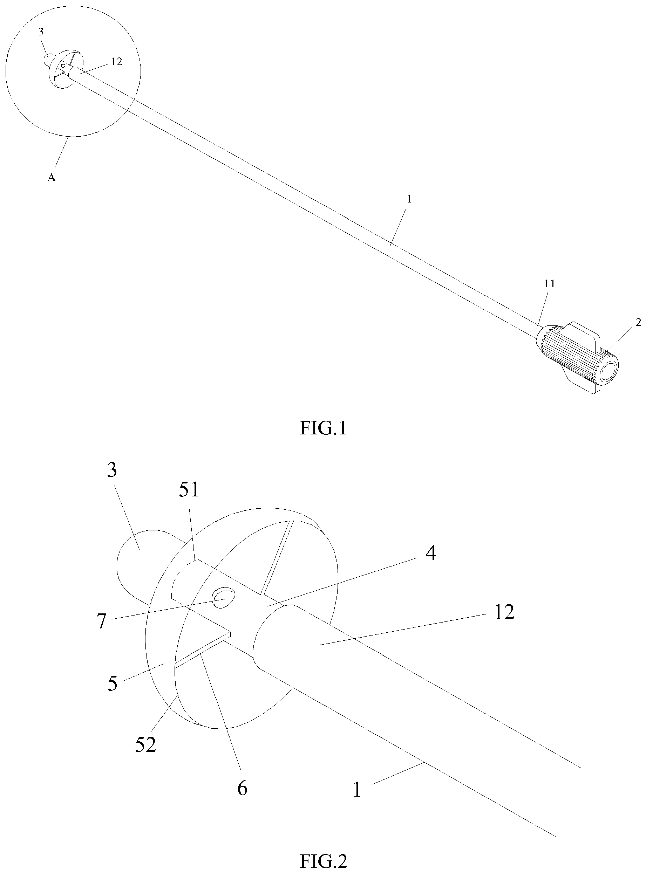

Referring to Figure 1, Figure 1 is a structure diagram of one embodiment of single valve film type ureteral stone blockage extractor. The ureteral stone blockage extractor is provided with an operating rod 1. The operating rod 1 is cylinder-shaped hollow tube with open proximal end and closed distal end. The periphery of the proximal end segments 11 of the operating rod 1 is provided with handle 2. The top end of the distal end segment 12 of the operating rod 1 is provided with hemispherical guiding tip 3.

Referring to Figure 2, Figure 2 is an enlarged cutaway view of A in Figure 1. A fitting groove 4 is provided at the back side of the guiding tip 3 of the distal end segment 12 of the operating rod 1. The fitting groove 4 surrounds the operating rod 1 for one circle. The fitting groove 4 is provided with an occluding film 5. The occluding film 5 is configured to expand as hemispherical shape under normal usage. The distal edge of the occluding film 5 surrounds and is fixed to the distal outer wall of the fitting groove 4, the distal edge is defined as the fixed side of the occluding film 51. The proximal edge of the occluding film 5, that is the opposite side to the occluding film 51 is defined as the free side of the occluding film 52. A distance from a free side of the occluding film 52 to the proximal end of the operating rod 1 is less than a distance from a fixed side of the occluding film 51 to the proximal end of the operating rod 1. There are 3 supporters 6 provided in between the occluding film 5 and the fitting groove 4. Each of the supporter 6 is sheet like structure, of which, the first side is fixed on the outer wall of the fitting groove 4 along the longitudinal axis of the operating rod 1, the second side is curved and fixed to the inner wall of the occluding film 5, the third side is perpendicular to the operating rod 1. The 3 supporters 6 is configured to surround the operating rod 1 and are radially configured with equal space intervals. There are provided through-holes 7 in a wall of the fitting groove 4. There are 3 through-holes 7 in total which are configured between adjacent supporters 6.

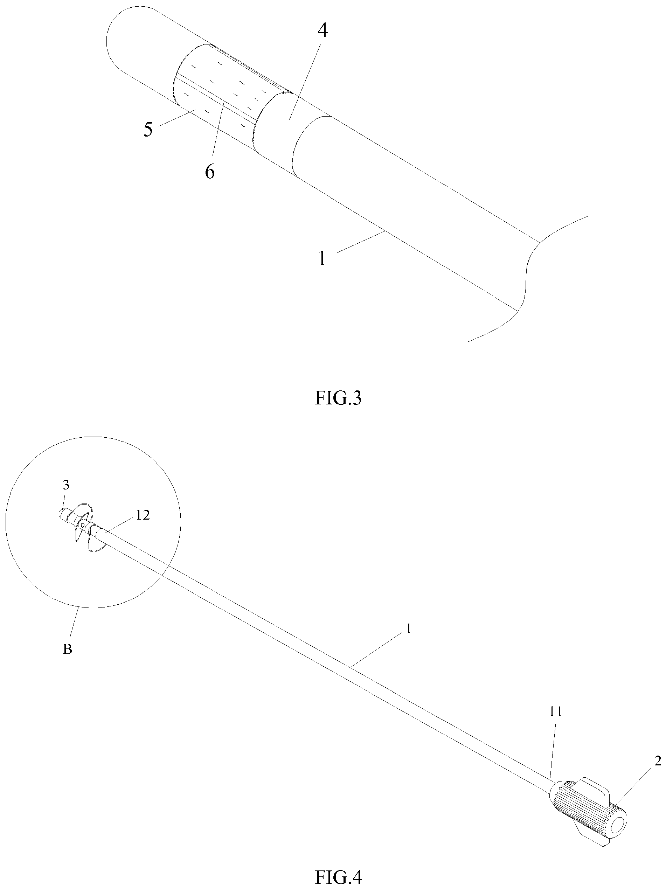

Referring to Figure 3, Figure 3 is a structure diagram of one embodiment of single valve film type ureteral stone blockage extractor before the extractor is used. The occluding film 5 and supporter 6 fold and affixed to the fitting groove 4 before the ureteral stone blockage extractor of the present invention is used, that is factory state, so that the diameter of fitting groove 4 is not larger than the diameter of the operating rod 1.

To be specified:
The occluding film 5 is used for occluding stone and cooperate with the operating rod 1 to extract stone. The occluding film 5 is of film shape, so the contact surface between the occluding film 5 and the inner wall of the ureter is very small, which will greatly reduce the incidence of stone fragment concentration in ureter. Furthermore, the occluding film 5 is soft, so the diameter of the occluding film 5 can be designed to be slightly larger than the diameter of the ureter. In one preferred embodiment, the diameter of the occluding film is between about 4mm and about 5mm, thus, the ureter lumen can be fully occluded to prevent stone from slipping and missing, stone can be extracted by one time, and the incidence of concentration can be reduced. The distance from a free side of the occluding film 52 to the proximal end of the operating rod 1 is less than the distance from a fixed side of the occluding film 51 to the proximal end of the operating rod 1, that is the entire occluding film 5 wraps up the distal end of the operating rod 1 like an umbrella. This kind of design can ensure that the occluding film 5 can maintain preformed shape and is not easy to turn over and deform when it was pushed both by the water stream through the working channel of endoscope and the water stream from the through-hole 7, thus no stone will slip or get concentrated. On the other side, the occluding film 5 with this kind of shape can form a space that can contain parts of stone fragments, thus minimize the friction between the stone fragments and ureter wall, and reduce injuries to the ureter wall. The shape of the occluding film 5 is not limited to hemispherical shape, it can also be any other shape that can ensure the curved outer surface of the occluding film 5, and the maximum cross sectional diameter is slightly larger than the diameter of the ureter. However hemispherical shape is the preferred shape which can ensure fully tightly attachment between the occluding film 5 and the inner wall of the ureter, thoroughly occlude stone and unavailable to deform under normal usage.

The supporter 6 is used to support and sustain the shape of the occluding film 5. The amounts and shape of supporter 6 is not limited to this embodiment, any amount and shape that can support the occluding film 5 can also be used. In one preferred embodiment, the supporter 6 surround the operating rod 1 and are radially configured with equal space intervals. In this case, the supporter 6 can provide equivalent supporting force. In another preferred embodiment, the supporter 6 is sheet like structure, of which, the first side is fixed on the outer wall of the fitting groove 4 along the longitudinal axis of the operating rod 1, the second side is curved and fixed to the inner wall of the occluding film 5, which will provide the most prominent supporting force.

The operating rod 1 is hollow with open proximal end. A through-hole 7 is provided in a wall of the fitting groove 4, thus water can be injected into the lumen of the operating rod 1, then streams of water flows out through the through-hole 7 to exert outward force on the inner wall of the occluding object 5. The force will neutralize the force exerted to the occluding film 5 that comes from the water stream infused through the working channel of the endoscope to prevent the occluding film 5, especially the free side of the occluding film 52 from turning over and maintain the shape of the occluding film 5 during the surgical procedure. The shape of the through-hole 7 is not restricted to annular shape, it can be any other shape that can form the water injecting channel, such as, for example, triangle shape. The configuration and location of the through-hole 7 can be in any manner that can exert outward force to the inner wall of the occluding film 5.

The material of the occluding film 5 and supporter 6 can be selected from TPU, Pebax, FEP, ETFE, TPFE etc. These kind of materials can be preformed and shrink and fold to a small volume, which can secure small volume of the extractor of the invention. Meanwhile, these kind of materials have good malleability and formability, is not easy to deform, and have resist capability for stone.

The fitting groove 4 is used for containing the occluding film 5 and supporter 6 before the extractor is used, so that the overall diameter of the extractor of the invention will be comparatively small, and the extractor can easily steer clear of the stone when being introduced. The extractor is also not easy to cause dislodge of stone or injure the inner wall of the ureter. The fitting groove 4 is not limited to be designed to surround the operating rod 1 by one circle. It can also be multiple stripped fitting grooves surround the operating rod 1 with interval configuration.

The end of the handle 2 is designed to match the end of the injector for conveniently injecting water. The guiding tip 3 is preferably designed as hemispherical or other curved shape that can guide the extractor of the present invention into the ureter without any injuries to ureter wall.

The guiding tip 3, occluding film 5 and supporter 6 can be independent elements, and they can be fixed on the operating rod 1 by welding, sticking, or other methods. They can also be one-batch formed together with the operating rod 1.

The exemplary use method of the ureteral stone blockage extractor includes:
when the ureteroscopic lithotripsy is being executed, the working channel of the endoscope shall be formed. Input the endoscope into the working channel, unfold the factory state package of the extractor, hold the handle 2 and input the guiding tip 3 of the operating rod 1 into the ureter, steer clear of the stone to get to the upper side of the stone. Then inject water into the lumen of the operating rod 1 through the proximal end of the operating rod 1. The injected water gets through the through-hole to push the inner wall of the occluding film 5. The occluding film 6 which will subsequently be extended and tightly attach to the inner wall of the ureter. Then infuse water through the working channel of the endoscope to the ureter, and inject water to the lumen of the operating rod 1 while dragging the operating rod 1 to the proximal end. Stone fragments will be flushed by continuous infused water into the space formed by the occluding film 6, or flow to the bladder while the water flushed and stirred and the operating rod 1 is slowly being dragged.

### Embodiment 2 Multiple valves film type ureteral stone blockage extractor

Referring to Figure 4, Figure 4 is a structure diagram of one embodiment of multiple valves film type ureteral stone blockage extractor. The ureteral stone blockage extractor is provided with operating rod 1. The operating rod 1 is cylinder-shaped hollow tube with open proximal end and closed distal end. The periphery of the proximal end segments of the operating rod 11 is provided with handle 2. The top end of the distal end segment of the operating rod 12 is provided with hemispherical guiding tip 3.

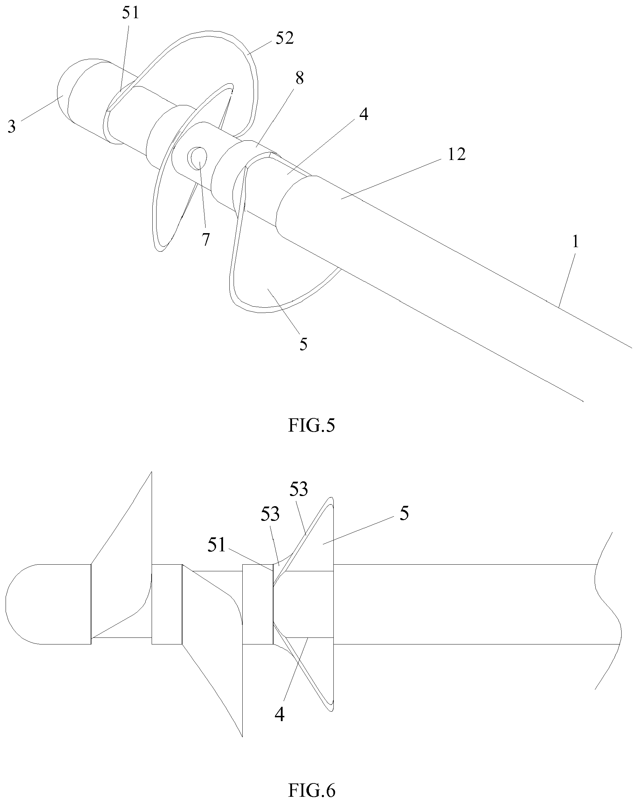

Referring to Figure 5, Figure 5 is an enlarged cutaway view of B in Figure 4. A fitting groove 4 is provided at the back side of the guiding tip 3 of the distal end segment of the operating rod 12. The fitting groove 4 surrounds the operating rod 1 by one circle. There are provided two annular reinforced film element 8 on the fitting groove 4. Both of the reinforced film element 8 surround the fitting groove 4 to separate the fitting groove 4 into three segments. Each segment of the fitting groove 4 is provided with an occluding film 5. The occluding film 5 expands as a fan with curved surface under normal usage. The distal edge of the three occluding films 5 are all fixed on the outer wall of the fitting groove 4, the distal edge is defined as the fixed side of the occluding film 51. Specifically, the fixed side of the occluding film 51 is separately fixed to the distal end of the fitting groove 4 or the proximal end of the reinforced film element 8. The proximal edge of the occluding film 5, that is the opposite side to the occluding film 51, is defined as the free side of the occluding film 52. A distance from a free side of the occluding film 52 to the proximal end of the operating rod 1 is less than a distance from a fixed side of the occluding film 51 to the proximal end of the operating rod 1. The three occluding films 5 is configured to surround the operating rod 1 in interlocked and overlapped arrangement. There is provided through-hole 7 in a wall of each segment of the fitting groove 4. The through-holes 7 are located on the both sides of the symmetry axis of the occluding films 5.

Referring to Figure 6, Figure 6 is the front view of a multiple valves film type ureteral stone blockage extractor. Each of the two lateral edges 53 of the occluding film 5 and the fixed side of the occluding film 51 form an included angle area 54, the two included angle areas 54 is close to each other, and clad to the wall of the fitting groove 4.

To be specified:
The occluding film 5 and supporter 6 fold and affixed to the fitting groove 4 before the ureteral stone blockage extractor is used, that is factory state, so that the diameter of fitting groove 4 is not larger than the diameter of the operating rod 1. The occluding film 5 is used for occluding stone and cooperate with the operating rod 1 to extract stone. The amount of the occluding film 5 is not limited to this embodiment, it can be 4, 5, 6 or other number that can ensure each of the occluding films 5 surround the operating rod 1 in interlocked and overlapped configuration, and each of the occluding films 5 can form a round occluding object without obvious gap when viewing from one end of the operating rod 1 to the other end thereof. The distance between adjacent occluding film 5 is set to be not allow stone to slip out and move reversely. The occluding film 5 is preformed to be preferably fan shape with curved surface, which will ensure that all the occluding film 5 can form a complete occluding structure without any gap. In one preferred embodiment, the cross sectional diameter of the occluding structure is preferably slightly larger than the diameter of the ureter. Preferably, the maximum cross sectional diameter of the occluding structure is specifically between about 4mm and about 5mm. The distance from a free side of the occluding film 52 to the proximal end of the operating rod 1 is less than the distance from a fixed side of the occluding film 51 to the proximal end of the operating rod 1, that is the entire occluding film 5 wraps up the distal end of the operating rod 1 like an umbrella. This kind of design can ensure that the occluding film 5 can maintain preformed shape and is not easy to turn over and deform when it was pushed both by the water stream through the working channel of endoscope and the water stream from the through-hole 7, thus no stone will slip or get concentrated. On the other side, the occluding film 5 with this kind of shape can form a space that can contain parts of stone fragments, thus minimize the friction between the stone fragments and ureter wall, and reduce injuries to the ureter wall. The two included angle areas 54 is close to each other, and clad to the wall of the fitting groove 4 to sustain the shape of the occluding film 5. Each of the distal edge of the occluding films 51 of the three occluding film 5 are fixed to the distal end of the fitting groove 4 or the proximal end of the reinforced film structure 8, thus the distal end of the fitting groove 4 or the proximal end of the reinforced film element 8 will restrain the free side of the occluding film 52 in case the occluding film 5 is turning over. The occluding film 5 is film, so the contact surface between the occluding film 5 and the inner wall of the ureter is very small, which will greatly reduce the incidence of fragment concentration in ureter. The material of the occluding film 5 can be selected from TPU, Pebax, FEP, ETFE, TPFE etc. These kind of materials can be shrunk and folded to a small volume, which can secure small volume of the extractor of the invention. Meanwhile, these kind of materials have good malleability and formability, is not easy to deform, and have resist capability for stone.

The operating rod 1 is hollow with open proximal end. A through-hole 7 is provided in a wall of the fitting groove 4, thus water can be injected into the lumen of the operating rod 1, then streams of water flows out through the through-hole 7 to exert outward force on the inner wall of the occluding object 5. The force will neutralize the force exerted to the occluding film 5 that comes from the water stream infused through the working channel of the endoscope to prevent the occluding film 5 from turning over and maintain the shape of the occluding film 5 during the surgical procedure. The shape of the through-hole 7 is not restricted to annular shape, it can be any other shape that can form the water injecting channel, such as, for example, triangle shape. The configuration and location of the through-hole 7 can be in any manner that can exert outward force to the inner wall of the occluding film 5. In another preferred embodiment, the through-holes 7 are located on the both sides of the symmetry axis of the occluding films 5, which will provide equivalent force to the occluding film 5.

The fitting groove 4 is used for containing the occluding film 5 before the extractor is used, so that the overall diameter of the extractor of the invention will be comparatively small, and the extractor can easily steer clear of the stone when being introduced. The extractor is also not easy to cause dislodge of stone or injure the inner wall of the ureter. The fitting groove 4 can be a complete segment, that is no reinforced film element 8 is provided. The fitting groove 4 is not limited to be designed to surround the operating rod 1 by one circle. It can also be multiple stripped fitting grooves surround the operating rod 1 with interval configuration.

The end of the handle 2 is designed to match the end of the injector for conveniently injecting water. The guiding tip 3 is preferably designed as hemispherical or other curved shape that can guide the extractor of the present invention into the ureter without any injuries to ureter wall.

The guiding tip 3 and occluding film 5 can be independent elements, and they can be fixed on the operating rod 1 by welding, sticking, or other methods. They can also be one-batch formed together with the operating rod 1.

The exemplary use method of the ureteral stone blockage extractor includes:
when the ureteroscopic lithotripsy is being executed, the working channel of the endoscope shall be formed. Input the endoscope into the working channel, unfold the factory state package of the extractor, hold the handle 2 and input the guiding tip 3 of the operating rod 1 into the ureter, steer clear of the stone to get to the upper side of the stone. Then inject water into the lumen of the operating rod 1 through the proximal end of the operating rod 1. The injected water gets through the through-hole to push the inner wall of the occluding film 5. The occluding film 6 which will subsequently be expanded. All the occluding film 5 will form a round occluding structure, the edge of the occluding structure is tightly attach to the inner wall of the ureter. Then infuse water through the working channel of the endoscope to the ureter, and inject water to the lumen of the operating rod 1 while dragging the operating rod 1 to the proximal end. Stone fragments will be flushed by continuous infused water into the space formed by the occluding film 6, or flow to the bladder while the water flushed and stirred and the operating rod 1 is slowly being dragged.

### Embodiment 3 Spiral film type ureteral stone blockage extractor

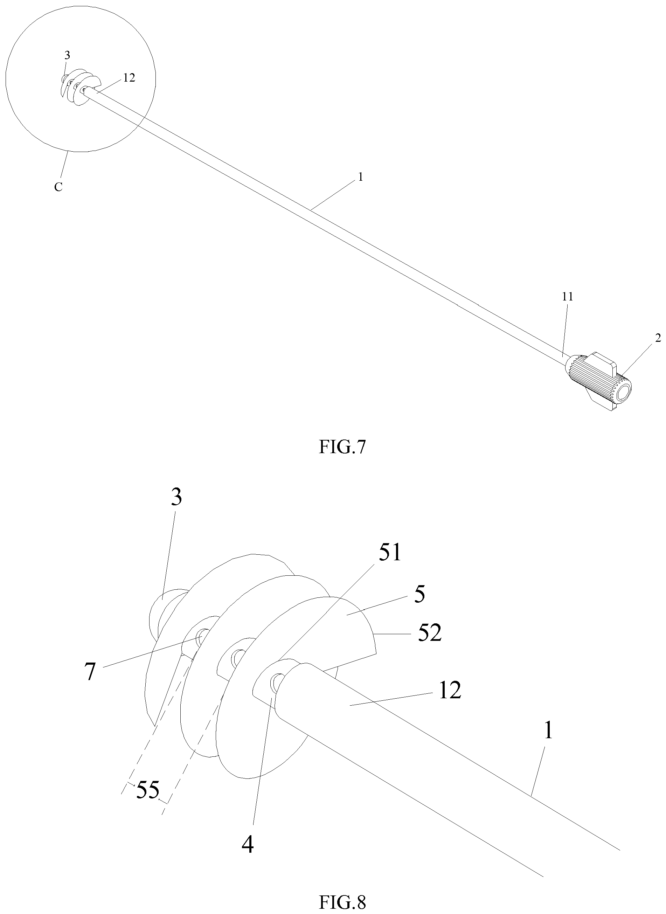

Referring to Figure 7, Figure 7 is a structure diagram of one embodiment of spiral film type ureteral stone blockage extractor. The ureteral stone blockage extractor is provided with operating rod 1. The operating rod 1 is cylinder-shaped hollow tube with open proximal end and closed distal end. The periphery of the proximal end segments of the operating rod 11 is provided with handle 2. The top end of the distal end segment of the operating rod 12 is provided with hemispherical guiding tip 3.

Referring to Figure 8, Figure 8 is an enlarged cutaway view of C in Figure 7. A fitting groove 4 is provided at the back side of the guiding tip 3 of the distal end segment of the operating rod 12. The fitting groove 4 surrounds the operating rod 1 by one circle. The fitting groove 4 is provided with an occluding film 5. The occluding film 5 is configured to expand as spiral shape, surrounds and is fixed to the wall of the fitting groove 4 by 3 circles under normal usage. The occluding film 5 surrounds the operating rod is defined as one occluding film sub unit 55, therefore there are 3 occluding film sub units 55. The distal edge of each of the occluding film sub unit 55 spirally surrounds and is fixed to the distal outer wall of the fitting groove 4. The distal edge of the occluding film sub unit 55 is defined as the fixed side of the occluding film 51. The proximal edge of the occluding film sub unit 55, that is the opposite side to the occluding film 51, is defined as the free side of the occluding film 52. A distance from a free side of the occluding film 52 to the proximal end of the operating rod 1 is less than a distance from a fixed side of the occluding film 51 to the proximal end of the operating rod 1. The diameter of each of the occluding film sub unit 55 is the same, that is viewing from the distal end to the proximal end of the operating rod 1, the edge of each of the occluding film sub unit 55 is overlapped, and the entire occluding film 5 configures as a cylinder occluding structure. There is provided through-hole 7 in a wall of the fitting groove 4 between adjacent occluding film sub unit 55.

To be specified:
The occluding film 5 fold and affixed to the fitting groove 4 before the ureteral stone blockage extractor is used, that is factory state, so that the diameter of fitting groove 4 is not larger than the diameter of the operating rod 1.

The occluding film 5 is used for occluding stone and cooperate with the operating rod 1 to extract stone. The occluding film 5 can be a one-piece film or comprise a plurality of films spirally configured, which can make sure the occluding films 5 can form a round occluding object without obvious gap when viewing from one end of the operating rod 1 to the other end thereof. Spiral shaped occluding film 5 can exert multiple blocking force to stone, which can effectively prevent stone from moving reversely. The distance between adjacent occluding film sub units 55 is not fixed. It can be any distance that not allow stone to slip out and move reversely. The diameter of each of the occluding film sub unit 55 is the same or different, that is viewing from the distal end to the proximal end of the operating rod 1, the edge of each of the occluding film sub unit 55 is overlapped or not overlapped, and the entire occluding film 5 configures as a cylinder shaped occluding structure or pillar shaped occluding structure. The maximum cross sectional diameter of the occluding structure 5 is preferably slightly larger than the diameter of the ureter. Preferably, the maximum cross sectional diameter of the occluding structure is specifically between about 4mm and 5mm. The distance from a free side of the occluding film 52 to the proximal end of the operating rod 1 is less than the distance from a fixed side of the occluding film 51 to the proximal end of the operating rod 1, that is the occluding film sub unit 55 has a dip angle relative to the operating rod 1. This kind of design can ensure that the occluding film 5 can maintain preformed shape and is not easy to turn over and deform when it was pushed both by the water stream through the working channel of endoscope and the water stream from the through-hole 7, thus no stone will slip or get concentrated. On the other side, the occluding film 5 with this kind of shape can form a space that can contain parts of stone fragments, thus minimize the friction between the stone fragments and ureter wall, and reduce injuries to the ureter wall. The occluding film 5 is film, so the contact surface between the occluding film 5 and the inner wall of the ureter is very small, which will greatly reduce the incidence of fragment concentration in ureter. The material of the occluding film 5 can be selected from TPU, Pebax, FEP, ETFE, TPFE etc. These kind of materials can be shrunk and folded to a small volume, which can secure small volume of the extractor of the invention. Meanwhile, these kind of materials have good malleability and formability, is not easy to deform, and have resist capability for stone.

The operating rod 1 is hollow with open proximal end. A through-hole 7 is provided in a wall of the fitting groove 4, thus water can be injected into the lumen of the operating rod 1, then streams of water flows out through the through-hole 7 to exert outward force on the inner wall of the occluding object 5. The force will neutralize the force exerted to the occluding film 5 that comes from the water stream infused through the working channel of the endoscope to prevent the occluding film 5, especially the free side of the occluding film 52 from turning over and maintain the shape of the occluding film 5 during the surgical procedure. The shape of the through-hole 7 is not restricted to annular shape, it can be any other shape that can form the water injecting channel, such as, for example, triangle shape. The configuration and location of the through-hole 7 can be in any manner that can exert outward force to the inner wall of the occluding film 5.

The fitting groove 4 is used for containing the occluding film 5 before the extractor is used, so that the overall diameter of the extractor of the invention will be comparatively small, and the extractor can easily steer clear of the stone when being introduced. The extractor is also not easy to cause dislodge of stone or injure the inner wall of the ureter. The fitting groove 4 is not limited to be designed to surround the operating rod 1 by one circle. It can also be multiple stripped fitting grooves surround the operating rod 1 with interval configuration.

The end of the handle 2 is designed to match the end of the injector for conveniently injecting water. The guiding tip 3 is preferably designed as hemispherical or other curved shape that can guide the extractor of the present invention into the ureter without any injuries to ureter wall.

The guiding tip 3 and occluding film 5 can be independent elements, and they can be fixed on the operating rod 1 by welding, sticking, or other methods. They can also be one-batch formed together with the operating rod 1.

The exemplary use method of the ureteral stone blockage extractor includes:
when the ureteroscopic lithotripsy is being executed, the working channel of the endoscope shall be formed. Input the endoscope into the working channel, unfold the factory state package of the extractor, hold the handle 2 and input the guiding tip 3 of the operating rod 1 into the ureter, steer clear of the stone to get to the upper side of the stone. Then inject water into the lumen of the operating rod 1 through the proximal end of the operating rod 1. The injected water gets through the through-hole to push the inner wall of the occluding film 5. The occluding film 6 which will subsequently be expanded. All the occluding film 5 will form a cylinder, pillar or other shaped occluding structure, the edge of the occluding structure is tightly attach to the inner wall of the ureter. Then infuse water through the working channel of the endoscope to the ureter, and inject water to the lumen of the operating rod 1 while dragging the operating rod 1 to the proximal end. Stone fragments will be flushed by continuous infused water into the space formed by the occluding film 6, or flow to the bladder while the water flushed and stirred and the operating rod 1 is slowly being dragged.

The ureteral stone blockage extractor of the present invention has a small dimension, can be easily introduced into a ureter, would not push a stone to move reversely towards a kidney and eliminate trauma to the ureter wall, can fully occlude the ureter, effectively prevents any stone and stone fragments from being flushed back into the kidney by injected liquid, is not prone to get stone stuck in ureter, does not deform easily or rub against the ureter wall during a stone extraction process, and can remove all stone in a single run, thus achieving the goal of efficient stone removal.

The ureteral stone blockage extractor of the present invention can not only be used for occlusion and removal of ureteral stone, but also for occlusion and removal within other kind of body lumens and other obstructions, including, but not limit to occlusion and removal of kidney stone, occlusion and removal of embolus of vessel, occlusion and stop of trocar puncture hole hemorrhage of vessel, occlusion and removal of obstructions in trachea or intestinal tract.

The above disclosed is only a preferred embodiment of the present invention example, of course should not be utilized to limit the range of rights of the present invention, ordinary skills in the art can make multiple improvements and changes should be enclosed in the scope of the appended claims.

## Claims

1. A film-type ureter stone blockage extractor comprising a hollow operating rod (1) that has an open proximal end and a closed distal end,
wherein,
an occluding film (5) is provided on a distal end segment of the operating rod (11);
during use of the extractor, viewing from one end of the operating rod to the other end thereof, the occluding film (5) is configured as a round occluding object;
a distance from a free side of the occluding film (52) to the proximal end of the operating rod (11) is less than a distance from a fixed side of the occluding film (51) to the proximal end of the operating rod (11);
a through-hole (7) is also provided in a wall of the operating rod (1), wherein water stream flowing from a lumen of the operating rod (1) through the through-hole (7) exerts force on an inner wall of the occluding object; and
supporters (6) are provided in between the occluding film (5) and the operating rod (1), the supporters (6) are attached to both an inner wall of the occluding film (5) and an outer wall of the operating rod (1) to support the occluding film (5).

2. The film-type ureter stone blockage extractor according to claim 1, the distal end of the operating rod (12) is provided with a fitting groove (4), the occluding film (5) is provided in the fitting groove (4), the through-hole (7) is provided on the wall of the fitting groove (4), the fitting groove (4) is used for containing the occluding film (5) and the supporters (6) before the extractor is used.

3. The film-type ureter stone blockage extractor according to claim 1 or 2, the occluding film (5) is a single piece of film, the outer surface of the occluding film (5) is curved when it is expanded under normal usage.

4. The film-type ureter stone blockage extractor according to claim 3, the supporters (6) are configured to surround the operating rod (1) and are radially configured with equal space intervals, the through-hole (7) is configured between adjacent supporters (6).

5. The film-type ureter stone blockage extractor according to claim 1 or 2, comprising a plurality of the occluding films (5), each of the occluding films (5) is configured to expand as a fan shape with curved surface during use of the extractor, and the occluding films (5) are configured to surround the operating rod (1) in interlocked and overlapped arrangement.

6. The film-type ureter stone blockage extractor according to claim 5, reinforced film element (8) is provided on the operating rod (1) in between the adjacent occluding films (5), the reinforced film element (8) surrounds the operating rod (1), and the fixed side of the occluding film (51) is fixed on the proximal end of the reinforced film element (8).

7. The film-type ureter stone blockage extractor according to claim 6, each of two lateral edges of the occluding film (5) and a fixed side of the occluding film (51) is arranged to form an included angle area, the two included angle areas are close to each other, and clad to the wall of the operating rod (1).

8. The film-type ureter stone blockage extractor according to claim 1, the occluding film (5) is configured to expand as spiral shape during use of the extractor, and to surround and to be fixed to the wall of the operating rod (1).

9. The film-type ureter stone blockage extractor according to claim 8, wherein the occluding film (5) surrounding the operating rod (1) by one circle is defined as one occluding film sub unit, all the sub units have the same or different diameters.

## Patentansprüche

1. Folienartiger Harnleitersteinblockade-Extraktor, umfassend eine hohle Betätigungsstange (1), die ein offenes proximales Ende und ein geschlossenes distales Ende aufweist, wobei
eine Okklusionsfolie (5) an einem distalen Endsegment der Betätigungsstange (11) vorgesehen ist;
während der Verwendung des Extraktors, bei Betrachtung von einem Ende der Betätigungsstange zu dem anderen Ende davon, die Okklusionsfolie (5) als ein rundes okkludierendes Objekt konfiguriert ist;
ein Abstand von einer freien Seite der Okklusionsfolie (52) zu dem proximalen Ende der Betätigungsstange (11) kleiner als ein Abstand von einer festen Seite der Okklusionsfolie (51) zu dem proximalen Ende der Betätigungsstange (11) ist;
ein Durchgangsloch (7) auch in einer Wand der Betätigungsstange (1) vorgesehen ist, wobei ein Wasserstrom, der von einem Lumen der Betätigungsstange (1) durch das Durchgangsloch (7) strömt, eine Kraft auf eine Innenwand des okkludierenden Objekts ausübt; und
Träger (6) zwischen der Okklusionsfolie (5) und der Betätigungsstange (1) vorgesehen sind, wobei die Träger (6) sowohl an einer inneren Wand der Okklusionsfolie (5) als auch an einer äußeren Wand der Betätigungsstange (1) befestigt sind, um die Okklusionsfolie (5) zu tragen.

2. Folienartiger Harnleitersteinblockade-Extraktor nach Anspruch 1, wobei das distale Ende der Betätigungsstange (12) mit einer Passnut (4) versehen ist, wobei die Okklusionsfolie (5) in der Passnut (4) vorgesehen ist, wobei das Durchgangsloch (7) an der Wand der Passnut (4) vorgesehen ist, wobei die Passnut (4) zum Aufnehmen der Okklusionsfolie (5) und der Träger (6) verwendet wird, bevor der Extraktor verwendet wird.

3. Folienartiger Harnleitersteinblockade-Extraktor nach Anspruch 1 oder 2, wobei die Okklusionsfolie (5) ein einzelnes Stück Folie ist, wobei die äußere Oberfläche der Okklusionsfolie (5) gekrümmt ist, wenn sie bei normaler Verwendung expandiert wird.

4. Folienartiger Harnleitersteinblockade-Extraktor nach Anspruch 3, wobei die Träger (6) konfiguriert sind, um die Betätigungsstange (1) zu umgeben, und radial mit gleichen Zwischenraumabständen konfiguriert sind, wobei das Durchgangsloch (7) zwischen benachbarten Trägern (6) konfiguriert ist.

5. Folienartiger Harnleitersteinblockade-Extraktor nach Anspruch 1 oder 2, umfassend eine Mehrzahl von Okklusionsfolien (5), wobei jede der Okklusionsfolien (5) konfiguriert ist, um während der Verwendung des Extraktors als eine Fächerform mit einer gekrümmten Oberfläche zu expandieren, und wobei die Okklusionsfolien (5) konfiguriert sind, um die Betätigungsstange (1) in verriegelter und überlappender Anordnung zu umgeben.

6. Folienartiger Harnleitersteinblockade-Extraktor nach Anspruch 5, wobei ein verstärktes Folienelement (8) an der Betätigungsstange (1) zwischen den benachbarten Okklusionsfolien (5) vorgesehen ist, wobei das verstärkte Folienelement (8) die Betätigungsstange (1) umgibt und wobei die feste Seite der der Okklusionsfolie (51) an dem proximalen Ende des verstärkten Folienelements (8) befestigt ist.

7. Folienartiger Harnleitersteinblockade-Extraktor nach Anspruch 6, wobei jede von zwei lateralen Kanten der Okklusionsfolie (5) und eine feste Seite der Okklusionsfolie (51) angeordnet sind, um einen eingeschlossenen Winkelbereich zu bilden, wobei die zwei eingeschlossenen Winkelbereiche nahe beieinander liegen und die Wand der Betätigungsstange (1) plattieren.

8. Folienartiger Harnleitersteinblockade-Extraktor nach Anspruch 1, wobei die Okklusionsfolie (5) konfiguriert ist, um während der Verwendung des Extraktors als eine Spiralform zu expandieren und um die Wand der Betätigungsstange (1) zu umgeben und an dieser befestigt zu werden.

9. Folienartiger Harnleitersteinblockade-Extraktor nach Anspruch 8, wobei die Okklusionsfolie (5), die die Betätigungsstange (1) durch einen Kreis umgibt, als eine Okklusionsfolien-Untereinheit definiert ist, wobei alle Untereinheiten den gleichen oder unterschiedliche Durchmesser aufweisen.

## Revendications

1. Appareil d'extraction de type membrane de calculs urétéraux d'obstruction comprenant une tige d'actionnement creuse (1) qui a une extrémité proximale ouverte et une extrémité distale fermée,
dans lequel,
un film d'occlusion (5) est ménagé sur un segment d'extrémité distale de la tige d'actionnement (11) ;
pendant l'utilisation de l'extracteur, en regardant depuis une extrémité de la tige d'actionnement à l'autre extrémité de celui-ci, le film d'occlusion (5) est configuré comme un objet d'occlusion circulaire ;
une distance depuis un côté libre du film d'occlusion (52) à l'extrémité proximale de la tige d'actionnement (11) est inférieure à une distance depuis un côté fixe du film d'occlusion (51) à une extrémité proximale de la tige d'actionnement (11) ;
un trou débouchant (7) est également ménagé dans une paroi de la tige d'actionnement (1),
dans lequel un courant d'eau s'écoulant d'une lumière de la tige d'actionnement (1) au travers du trou débouchant (7) exerce une force sur une paroi interne de l'objet d'occlusion ; et
des soutiens (6) sont ménagés entre le film d'occlusion (5) et la tige d'actionnement (1), les soutiens (6) sont fixés à la fois à une paroi intérieure du film d'occlusion (5) et une paroi extérieure de la tige d'actionnement (1) pour supporter le film d'occlusion (5).

2. Appareil d'extraction de type membrane de calculs urétéraux d'obstruction selon la revendication 1, l'extrémité distale de la tige d'actionnement (12) est dotée d'une rainure de montage (4),
le film d'occlusion (5) est ménagé dans la rainure de montage (4), le trou débouchant (7) est ménagé sur la paroi de la rainure de montage (4), la rainure de montage (4) est utilisée pour contenir le film d'occlusion (5) et les soutiens (6) avant que l'extracteur soit utilisé.

3. Appareil d'extraction de type membrane de calculs urétéraux d'obstruction selon la revendication 1 ou 2, le film d'occlusion (5) est un morceau unique de film, la surface extérieure du film d'occlusion (5) est courbée lorsqu'il est déployé lors d'une utilisation normale.

4. Appareil d'extraction de type membrane de calculs urétéraux d'obstruction selon la revendication 3, les soutiens (6) sont configurés pour entourer la tige d'actionnement (1) et sont configurés radialement avec des intervalles d'espacement égaux, le trou débouchant (7) est configuré entre des soutiens adjacents (6).

5. Appareil d'extraction de type membrane de calculs urétéraux d'obstruction selon la revendication 1 ou 2,
comprenant une pluralité de films d'occlusion (5), chacun des films d'occlusion (5) est configuré pour se déployer en forme d'éventail avec une surface courbée pendant une utilisation de l'extracteur, et les films d'occlusion (5) sont configurés pour entourer la tige d'actionnement (1) dans un agencement imbriqué et superposé.

6. Appareil d'extraction de type membrane de calculs urétéraux d'obstruction selon la revendication 5, un élément de film renforcé (8) est ménagé sur la tige d'actionnement (1) entre les films d'occlusion adjacents (5), l'élément de film renforcé (8) entoure la tige d'actionnement (1), et le côté fixé du film d'occlusion (51) est fixé sur l'extrémité proximale de l'élément de film renforcé (8).

7. Appareil d'extraction de type membrane de calculs urétéraux d'obstruction selon la revendication 6, chacun des deux bords latéraux du film d'occlusion (5) et d'un côté fixé du film d'occlusion (51) est agencé pour former une zone d'angle inclus, les deux zones d'angle inclus sont proches l'une de l'autre, et appliquées à la paroi de la tige d'actionnement (1).

8. Appareil d'extraction de type membrane de calculs urétéraux d'obstruction selon la revendication 1, le film d'occlusion (5) est configuré pour se déployer en forme de spirale pendant l'utilisation de l'extracteur, et pour entourer et être fixé à la paroi de la tige d'actionnement (1).

9. Appareil d'extraction de type membrane de calculs urétéraux d'obstruction selon la revendication 8,
dans lequel le film d'occlusion (5) entourant la tige d'actionnement (1) par un cercle est défini en tant que sous-unité de film d'occlusion, toutes les sous-unités ont le même diamètre ou des diamètres différents.
